# EUROPÄISCHE PATENTSCHRIFT

(11) **EP 4 115 824 B1**
(45) Veröffentlichungstag und Bekanntmachung des Hinweises auf die Patenterteilung: **25.09.2024**
(21) Anmeldenummer: 22000177.0
(22) Anmeldetag: 05.07.2022
(51) Int. Cl.: A61B 17/30, A61B 17/22, A61B 17/29, A61B 17/221, A61B 17/34, A61B 17/322, A61F 2/00

(54) **MANDRIN ZUM TRANSPLANTIEREN EINES HAUTFADENS**
STYLET FOR GRAFTING A SKIN FILAMENT
MANDRINE POUR GREFFER UN FIL CUTANÉ

(30) Priorität: 06.07.2021 DE 102021003482
(43) Veröffentlichungstag der Anmeldung: 11.01.2023
(73) Patentinhaber: Barth, Georg, 81679 München (DE)
(72) Erfinder: Barth, Georg, 81679 München (DE)

(56) Entgegenhaltungen:
- WO-A1-2012/093094
- US-A- 5 716 367
- US-A1- 2002 103 498
- US-A1- 2003 120 307
- US-A1- 2013 225 900
- US-A1- 2017 007 277
- US-A1- 2019 336 124
- US-A1- 2021 128 126
- US-B1- 10 092 168
- US-B1- 6 228 023

## Beschreibung

Die Erfindung betrifft einen Mandrin. Hierbei weist ein Grundkörper des Mandrins eine Eigensteifigkeit auf, welche ein Hindurchschieben des Grundkörpers durch einen Katheter zulässt. Der Mandrin weist einen ersten Endbereich auf, in welchem der Mandrin bei einem Zurückziehen des Mandrins greifbar ist. Durch das Zurückziehen des Mandrins ist eine Bewegung des Grundkörpers durch den Katheter hindurch in eine Richtung bewirkbar, welche einer Richtung bei dem Hindurchschieben entgegengesetzt ist.

Die US 5 716 367 A beschreibt eine Katheteranordnung zum intrakardialen Nähen.

Die US 6 228 023 B1 beschreibt einen Gewebegreifer zur Verwendung bei minimalinvasiven Operationen.

Die EP 1 601 407 B1 beschreibt ein System, welches einen flexiblen, röhrenförmigen Infusionskatheter und eine hohle Röhre umfasst. Die hohle Röhre ist steifer als der Infusionskatheter und fungiert als Mandrin, um den Katheter in einem Gewebe zu einem Zielort zu führen. Der Hohlraum der hohlen Röhre beziehungsweise des Mandrins ist mit einem Fluid gefüllt. Wenn der Infusionskatheter mittels des Mandrins zu seinem Zielort geführt ist, wird das Fluid aus der hohlen Röhre abgelassen. Auf diese Weise soll verhindert werden, dass nach einem Herausziehen des Mandrins aus dem Infusionskatheter in dem Infusionskatheter Luft verbleibt.

Mittels der hohlen Röhre lässt sich somit zwar ein Fluid an einen gewünschten Zielort verbringen. Jedoch ist die hohle Röhre beziehungsweise der Mandrin nicht zum Transplantieren von festem Gewebe wie etwa einem Hautfaden geeignet.

Als Folge von Verletzungen, Narben, Entzündungen und Alterung können beim Menschen Hautdefekte und Gewebedefekte auftreten. Infolge der Alterung der Haut kommt es diesbezüglich insbesondere zur einer Ausbildung von Falten. Ein Auffüllen von Hautdefekten oder Gewebedefekten ist im medizinischen und ästhetischen Bereich häufig Gegenstand von Prozeduren und Operationen. Wenn zur Auffüllung von Gewebedefekten wie etwa Falten weiche oder flüssige Substanzen verwendet werden, beispielsweise Eigenfett oder Hyaluronsäure, also ein Bestandteil des menschlichen Bindegewebes, so werden für derartige Transplantationen beziehungsweise Implantationen üblicherweise Spritzen verwendet.

Injektionen von flüssigem Material haben jedoch den Nachteil, dass das Material durch das sich bewegende Gewebe, etwa aufgrund mimischer Bewegungen im Gesicht, verschoben oder weggedrückt werden kann. Des Weiteren können flüssige oder weiche Substanzen im Körper resorbiert werden, so dass eine dauerhafte Auffüllung des Defekts oft nicht erreichbar ist. Darüber hinaus kommt es bei der Verwendung eines flüssigen körperfremden Materials etwa in Form von Hyaluronsäure zuweilen zu Abstoßungsreaktionen.

Es besteht auch die Möglichkeit, synthetische Fäden wie etwa Gore-Tex^{®} - Fäden oder Goldfäden zur Implantation zu verwenden, wobei zum Vernähen der Fäden eine Nadel zum Einsatz kommen kann. Auch bei derartigen Fäden aus körperfremdem Material kann es jedoch zu einer Abstoßung kommen. Und chronische Abstoßungsreaktionen können zu narbigen Funktionsausfällen beziehungsweise Entstellungen führen.

Folglich ist es wünschenswert, zum Auffüllen oder Ausgleichen von Gewebedefekten feste körpereigene Gewebearten, etwa in Form von korialen, also aus der menschlichen Lederhaut gewonnenen Hautfäden zu verwenden.

Die DE 42 11 889 A1 beschreibt in diesem Zusammenhang ein chirurgisches Instrument zur Transplantation beziehungsweise Implantation von Gewebe, wie etwa Korium, Fett oder Knorpel. Das chirurgische Instrument besteht aus einer steifen Kanüle mit drei Bereichen, welche an Trennstellen voneinander abgetrennt werden können. Nach einem Einbringen des Instruments in einen Hautbereich können die beiden Endbereiche an den Trennstellen abgebrochen werden, sodass zwei mit Gewebe gefüllte Halbschalen eines Mittelteils des Instruments vor Ort verbleiben. Anschließend sollen die beiden nach dem Abbrechen der Endbereiche der Kanüle voneinander getrennten Halbschalen aus dem Hautbereich herausgezogen werden.

Als nachteilig erweist sich hierbei, dass nur sehr schwer vermieden werden kann, beim Herausziehen der Halbschalen aus dem Hautbereich das zu transplantierende Gewebe mit herauszuziehen oder zu verschieben. Mittels des in der DE 42 11 889 A1 beschriebenen chirurgischen Instruments lässt sich somit eine Transplantation eines Hautfadens nicht auf zufriedenstellende Art und Weise erreichen.

Aufgabe der vorliegenden Erfindung ist es, einen Mandrin zu schaffen, welcher ein besonders einfaches Transplantieren eines Hautfadens ermöglicht.

Diese Aufgabe wird durch einen Mandrin mit den Merkmalen des Patentanspruchs 1 gelöst. Vorteilhafte Ausgestaltungen mit zweckmäßigen Weiterbildungen der Erfindung sind in den abhängigen Patentansprüchen und in der nachfolgenden Beschreibung angegeben.

Der erfindungsgemäße Mandrin ist zum Transplantieren eines Hautfadens ausgebildet. Ein Grundkörper des Mandrins weist eine Eigensteifigkeit auf, welcher ein Hindurchschieben des Grundkörpers durch einen peripheren Venenkatheter zulässt. Der Mandrin weist einen ersten Endbereich auf, in welchem der Mandrin bei einem Zurückziehen des Mandrins greifbar ist. Durch den ersten Endbereich ist ein Anschlag für das Hindurchschieben des Grundkörpers durch den Venenkatheter bereitgestellt. Durch das Zurückziehen des Mandrins ist eine Bewegung des Grundkörpers durch den Venenkatheter hindurch in eine Richtung bewirkbar, welche einer Richtung bei dem Hindurchschieben entgegengesetzt ist. Der Mandrin weist zumindest in einem zweiten Endbereich wenigstens ein Halteelement auf. Das wenigstens eine Halteelement ist dazu ausgebildet, während des Zurückziehens des Grundkörpers durch den Venenkatheter hindurch den zu transplantierenden Hautfaden festzuhalten.

Das Halteelement umfasst wenigstens zwei Teilstücke des Grundkörpers, welche zumindest in dem zweiten Endbereich des Mandrins voneinander weg bewegbar sind. Hierbei ist ein zwischen den beiden Teilstücken anordenbarer Teilbereich des Hautfadens aufgrund des Zurückziehens des Grundkörpers durch den Venenkatheter hindurch einklemmbar. Durch ein derartiges Auflösen des Grundkörpers zumindest in dem zweiten Endbereich in die wenigstens zwei Teilstücke lässt sich das Halteelement besonders einfach bereitstellen. Zudem lässt sich bei einer derartigen Ausgestaltung des Halteelements auch das Hindurchschieben des Grundkörpers durch den Venenkatheter hindurch derart, dass der zweite Endbereich des Mandrins an einem dem ersten Endbereich des Mandrins gegenüberliegenden Ende des Venenkatheters über das Ende des Venenkatheters übersteht oder aus dem Venenkatheter heraussteht, besonders einfach und zuverlässig realisieren.

Bei dem erfindungsgemäße Mandrin ist der Grundkörper über seine gesamte Länge in die wenigstens zwei Teilstücke getrennt. Hierbei sind die wenigstens zwei Teilstücke voneinander separat ausgebildet und zum Festhalten des Hautfadens voneinander weg bewegbar. Des Weiteren sind bei dieser Ausgestaltung des Halteelements die wenigstens zwei voneinander separaten Teilstücke mittels eines Kopplungselements des Mandrins zusammengehalten. Diese Ausgestaltung bringt es in vorteilhafterweise mit sich, dass nach einem Entfernen des Kopplungselements von den wenigstens zwei Teilstücken jedes einzelne der Teilstücke separat aus dem Venenkatheter oder dergleichen schützender Hülle herausgezogen werden kann. Folglich lässt sich sehr einfach sicherstellen, dass bei dem Herausziehen des Grundkörpers aus dem Venenkatheter der zu transplantierende Hautfaden in dem Venenkatheter verbleibt.

Die vorstehend genannten Vorteile gelten in besonderem Maße, wenn der Grundkörper über seine gesamte Länge in mehr als zwei Teilstücke getrennt oder gespalten ist.

Auf diese Weise ist es möglich, den zu transplantierenden Hautfaden durch eine Hülle in Form des Venenkatheters geschützt an seinen Zielort im aufzufüllenden oder auszugleichenden Gewebe zu verbringen. Wenn der Hautfaden dann an der passenden Stelle in dem Gewebe platziert ist, kann der Hautfaden beispielsweise in einem dem Halteelement des Mandrins gegenüberliegenden Endbereich festgehalten werden, während der den Hautfaden umgebende oder schützende Venenkatheter aus dem Gewebe entfernt wird. Der Hautfaden kommt dadurch in dem Gewebe zu liegen, welches durch das Transplantieren des wenigstens einen Hautfadens aufzufüllen ist. Da es sich bei dem Hautfaden um körpereigenes Gewebe handelt, kann der Hautfaden an der gewünschten Stelle gut einheilen. Zudem ist eine Verschiebung des Hautfadens nach dessen Einbringung in das aufzufüllende Gewebe an der gewünschten Stelle besonders weitgehend vermieden. Denn während des Einbringens des Hautfadens an die gewünschte Stelle in dem Gewebe ist der Hautfaden durch die Hülle in Form des Venenkatheters geschützt.

Das wenigstens eine Halteelement des Mandrins kann nach dem Hindurchschieben des Grundkörpers durch den Venenkatheter derart, dass das Halteelement an einem dem ersten Endbereich des Mandrins gegenüberliegenden Ende des Venenkatheters aus dem Venenkatheter heraustritt, zum Festhalten des zu transplantierenden Hautfadens verwendet werden. In dem aufzufüllenden Gewebe ist dann beim anschließenden Zurückziehen des Mandrins und somit auch bei dem Hindurchziehen des mittels des wenigstens einen Halteelements festgehaltenen Hautfadens durch den Venenkatheter hindurch der Hautfaden gut geschützt. Dadurch kann erreicht werden, dass der zu transplantierende Hautfaden unbeeinträchtigt an die Stelle des Gewebes gelangt, an welcher ein anschließendes Einheilen des Hautfadens in das Gewebe gewünscht ist. Folglich ermöglicht der Mandrin ein besonders einfaches Transplantieren des Hautfadens.

Dem einfachen Transplantieren des Hautfadens ist es außerdem zuträglich, dass durch den ersten Endbereich des Mandrins der Anschlag bereitgestellt ist, welcher die Bewegung des Mandrins bei dem Hindurchschieben des Grundkörpers durch den Venenkatheter hindurch begrenzt. Denn der erste Endbereich verhindert, dass der gesamte Mandrin in den Venenkatheter hineingeschoben werden kann. Der erste Endbereich weist hierfür vorzugsweise größere Abmessungen in radialer Richtung auf als der Grundkörper des Mandrins. Beispielsweise kann der erste Endbereich einen größeren Durchmesser aufweisen als der Grundkörper. Insbesondere sind die Abmessungen des ersten Endbereichs in radialer Richtung größer als ein Innendurchmesser des peripheren Venenkatheters. Auf diese Weise ist durch den ersten Endbereich ein besonders wirksamer Anschlag bereitgestellt.

Zudem lässt sich der Mandrin in diesem ersten Endbereich gut greifen und handhaben. Letzteres gilt insbesondere, wenn der Grundkörper des in dem ersten Endbereich gegriffenen Mandrins in eine Rotationsbewegung versetzt wird, während der Grundkörper durch den Venenkatheter hindurchgeschoben wird.

Der Mandrin ist als medizinisches Gerät ausgebildet und somit insbesondere zum Transplantieren oder Implantieren eines korialen Hautfadens in ein Gewebe in Form des Koriums beziehungsweise der Lederhaut der menschlichen Haut geeignet. Bei einer derartigen Verwendung des Mandrins kommt der koriale Hautfaden nach dem Entfernen des Venenkatheters im straffen Korium zu liegen. Hier liegt ein gut durchblutetes und eng an dem Hautfaden anliegendes Wundbett vor. Zudem handelt es sich bei dem korialen Hautfaden um ein im Ganzen vorliegendes, also nicht fraktioniertes Transplantat. Beide Umstände sind einer guten Einheilung des Hautfadens in dem Korium zuträglich.

Der Mandrin ist zwar insbesondere zum Transplantieren von korialen Hautfäden geeignet. Jedoch sind mittels des Mandrins auch andere körpereigene Gewebearten transplantierbar, welche ein Festgehaltenwerden mittels des wenigstens einen Halteelements des Mandrins zulassen. Dadurch lassen sich mittels des Mandrins Gewebedefekte der Haut besonders gut und nachhaltig auffüllen beziehungsweise ausgleichen.

Des Weiteren bringt es die Eignung des Grundkörpers, durch den peripheren Venenkatheter oder Venenverweilkatheter hindurchgeschoben zu werden, mit sich, dass der Grundkörper auch durch eine andersartige schützende Hülle, etwa in Form einer aus einem flexiblen Material gebildeten Kanüle oder dergleichen hindurchgeschoben werden kann.

Gängige oder handelsübliche periphere Venenkatheter zur Anwendung beim Menschen weisen insbesondere Außendurchmesser im Bereich von 0,7 mm bis 2,2 mm und entsprechende Innendurchmesser im Bereich von 0,4 mm bis 1,7 mm auf. Längen beziehungsweise Stichlängen derartiger Venenverweilkatheter liegen im Bereich von etwa zwei Zentimetern bis etwa fünf Zentimetern.

Der Mandrin ist vorliegend derart ausgebildet, dass der Grundkörper des Mandrins insbesondere durch einen derartigen peripheren Venenkatheter oder Venenverweilkatheter mit den genannten Abmessungen hindurchgeschoben werden kann. Auf diese Weise kann nämlich sichergestellt werden, dass durch das Einbringen des Venenkatheters in das Gewebe, in welches der Hautfaden transplantiert beziehungsweise implantiert werden soll, keine unerwünscht starke Verletzung des Gewebes auftritt. Der Grundkörper des Mandrins kann jedoch auch derart bemessen sein, dass der Grundkörper zusammen mit dem wenigstens einen Halteelement durch einen peripheren Venenkatheter oder Venenverweilkatheter mit einem Innendurchmesser von bis zu etwa 5 mm hindurchgeschoben werden kann.

Des Weiteren ist die Eigensteifigkeit des Grundkörpers des Mandrins zwar ausreichend groß, um das Hindurchschieben des Grundkörpers durch den peripheren Venenkatheter zuzulassen. Vorzugsweise ist der Grundkörper jedoch ausreichend flexibel, um beim Hindurchschieben durch den peripheren Venenkatheter (oder durch eine von den Abmessungen gleichartige schützende Hülle) hindurch möglicherweise vorhandenen Krümmungen des Venenkatheters (beziehungsweise der Hülle) folgen zu können. Dadurch lässt sich mittels des Mandrins in einer Vielzahl von aufzufüllenden oder auszugleichenden Gewebebereichen eine Transplantation von Hautfäden vornehmen.

Von Vorteil ist es, wenn der Mandrin in dem ersten Endbereich eine größere Rauheit aufweist als eine Oberfläche des Grundkörpers, welche einer Innenseite des Venenkatheters zugewandt ist, insbesondere beim Hindurchschieben des Grundkörpers durch den Venenkatheter hindurch an dieser Innenseite anliegt. Denn dann lässt sich der Mandrin selbst dann gut und sicher handhaben, wenn der Mandrin in dem ersten Endbereich mit einem Handschuh gegriffen wird.

Ein Vorteil der Verwendung von körpereigenen Transplantaten, insbesondere in Form des aus korialem Gewebe gewonnenen Hautfadens, zum Auffüllen oder Ausgleichen Hautdefekten von besteht insbesondere darin, dass durch Einwachsen eines solchen Transplantats an der richtigen Stelle der Hautdefekt dauerhaft aufgefüllt werden kann, ohne dass Abstoßungsreaktionen ausgelöst werden.

Prinzipiell ist es mittels des zum Transplantieren des Hautfadens geeigneten Mandrins jedoch auch möglich, körperfremde Materialien, etwa in Form eines Spendergewebes eines anderen Menschen oder eines Gewebes einer anderen Spezies, zu transplantieren. Des Weiteren können auf die beispielhaft für den Hautfaden geschilderte Art und Weise substanzabgebende beziehungsweise substanzaufnehmende Transplantate an eine gewünschte Stelle verbracht werden, wobei es sich bei der wenigstens einen Substanz insbesondere um ein Medikament oder dergleichen handeln kann. Außerdem ist es möglich, auf diese Weise strahlenabgebende Elemente und/oder strahlenaufnehmende Elemente an einer gewünschten Stelle im Gewebe zu platzieren. Letzteres kann insbesondere im Zusammenhang mit einer Strahlentherapie vorteilhaft genutzt werden.

Wenn das Halteelement genau zwei Teilstücke des Grundkörpers umfasst, so ist das Halteelement nach Art einer Pinzette ausgebildet, deren Arme die beiden Teilstücke bilden und somit zum Ergreifen des Teilbereichs des Hautfadens voneinander weg bewegbar sind. Wird der zwischen den Armen oder Teilstücken angeordnete Teilbereich des Hautfadens dann zusammen mit dem Grundkörper zurückgezogen, bewirkt der lichte Querschnitt oder Innendurchmesser des Venenkatheters das Einklemmen des Teilbereichs des Hautfadens zwischen den Armen oder Teilstücken des Grundkörpers. Dies ist dem einfachen Transplantieren des Hautfadens zuträglich.

Es ist jedoch auch möglich, den Grundkörper zumindest in dem zweiten Endbereich in mehr als zwei Teilstücke aufzulösen oder zu spalten und so das Halteelement bereitzustellen. Auf diese Weise lässt sich ein sehr behutsames und dennoch sicheres Festhalten des Hautfadens während des Zurückziehens des Grundkörpers durch den Venenkatheter hindurch erreichen. Denn insbesondere lässt sich so der zu haltende Teilbereich des Hautfadens sehr umfassend einklemmen beziehungsweise besonders weitgehend umschließen.

Die wenigstens zwei Teilstücke des Grundkörpers können in einer nicht unter den Patentanspruch 1 fallenden Variante des Mandrins in einem Verbindungsbereich miteinander verbunden sein. So ist auf besonders einfache Art und Weise sichergestellt, dass bei dem voneinander weg Bewegen der Teilstücke zwar ein Aufweiten des Halteelements stattfindet, jedoch die wenigstens zwei Teilstücke in dem Verbindungsbereich miteinander verbunden bleiben. Dies ist im Hinblick auf eine einfache Handhabung des Mandrins vorteilhaft.

Der Verbindungsbereich kann zwischen dem ersten Endbereich des Mandrins und jeweiligen freien oder distalen Enden der wenigstens zwei Teilstücke ausgebildet sein. Je nachdem, wie weit der Verbindungsbereich von den freien oder distalen Enden der wenigstens zwei Teilstücke beabstandet ist, kann das Halteelement mehr oder weniger stark aufgeweitet werden.

Des Weiteren kann bei dieser Variante des Mandrins vorgesehen sein, dass die wenigstens zwei Teilstücke an einem sich Längsrichtung des Grundkörpers erstreckenden Randbereich der Teilstücke miteinander verbunden sind. So kann durch ein Aufklappen der Teilstücke um eine Schwenkachse, welche sich in eine axiale Richtung des länglichen Grundkörpers erstreckt, das Halteelement in eine Offenstellung verbracht werden, in welcher der Hautfaden beziehungsweise das zu transplantierende Gewebe zwischen die beiden Teilstücke eingelegt werden kann. Indem die beiden Teilstücke in dem Randbereich miteinander verbunden sind, ist der Zusammenhalt der Teilstücke sichergestellt. Auch dies ist im Hinblick auf eine einfach Handhabung des Mandrins beim Festhalten des Hautfadens und beim Zurückziehen des Grundkörpers durch den Venenkatheter oder eine derartige schützende Hülle hindurch vorteilhaft.

Vorzugsweise sind die voneinander separaten Teilstücke in dem ersten Endbereich des Mandrins mittels des Kopplungselements zusammengehalten. Denn dies geht mit einer guten Zugänglichkeit zu dem Kopplungselement einher.

Das Kopplungselement kann insbesondere nach Art einer aus einem elastischen Material gebildeten Manschette oder Hülse ausgebildet sein, welches die voneinander separat ausgebildeten Teilstücke des Grundkörpers zusammenhält.

Vorzugsweise begrenzen die wenigstens zwei Teilstücke in einer Haltestellung, in welcher das Einklemmen des Teilbereichs des Hautfadens mittels des Halteelements bewirkbar ist, einen in dem Grundkörper ausgebildeten Aufnahmeraum. So ist ein zugleich wirksames und dennoch schonendes Festhalten des Teilbereichs des Hautfadens durch das Einklemmen desselben erreichbar. Beispielsweise können zu diesem Zweck die Teilstücke nach Art von Halbschalen ausgebildet sein, welche im Querschnitt eine gekrümmte, insbesondere kreisbogenförmige Gestalt aufweisen. Auf diese Weise ist eine besonders gute Druckverteilung beim Einklemmen des Teilbereichs des Hautfadens in der Haltestellung des Halteelements erreichbar.

Als weiter vorteilhaft hat es sich gezeigt, wenn die wenigstens zwei Teilstücke in der Haltestellung jeweils eine dem Teilbereich des Hautfadens zugewandte Halteseite und eine der Halteseite abgewandte Außenseite aufweisen, wobei eine Rauheit der Halteseite größer oder gleich einer Rauheit der Außenseite ist. Durch eine entsprechende Rauheit der Halteseite kann ein besonders gutes Festhalten des Teilbereichs des Hautfadens erreicht werden. Insofern ist es günstig, wenn die Rauheit der Halteseite mindestens gleich der Rauheit der Außenseite ist, wobei die Rauheit der Halteseite vorzugsweise größer als die Rauheit der Außenseite ist.

Die Rauheit der Außenseite der wenigstens zwei Teilstücke ist vorzugsweise derart bemessen, dass ein besonders ungehindertes Zurückziehen des Grundkörpers durch den Venenkatheter hindurch in die Richtung erreicht werden kann, welche der Richtung bei dem Hindurchschieben des Grundkörpers durch den Venenkatheter hindurch entgegengesetzt ist.

Ein zu großer Reibwert beziehungsweise eine zu große Rauheit der Halteseite ist jedoch vorzugsweise zu vermeiden. Denn dann kann ein Lösen des Hautfadens von dem Halteelement sehr einfach erreicht werden, etwa indem auf das distale beziehungsweise freie Ende des Hautfadens, welches dem festgehaltenen Teilbereich des Hautfadens gegenüberliegt, ein Gegenzug aufgebracht wird.

An der Halteseite kann eine gewünschte Rauheit des Teilstücks insbesondere durch Vorsehen eines Silikonmaterials bereitgestellt werden, etwa durch Aufbringen einer Beschichtung aus dem Silikonmaterial auf einen Trägerkörper des jeweiligen Teilstücks oder durch Ausbilden zumindest eines Teilbereichs des Halteelements aus einem Silikonmaterial.

Wenn der Grundkörper sich zu dem zweiten Endbereich hin verjüngend ausgebildet ist, so erleichtert dies das Hindurchschieben des Grundkörpers durch den Venenkatheter. Beispielsweise kann hierfür der Grundkörper zu dem freien Endbereich oder distalen Ende des Mandrins hin konisch zulaufen.

Als weiter vorteilhaft hat es sich gezeigt, wenn der Grundkörper zumindest im Bereich des Halteelements transparent ausgebildet ist. Denn dann kann mit dem menschlichen Auge gut erkannt werden, ob der Teilbereich des zu transplantierenden Hautfadens wie gewünscht im Bereich des Halteelements angeordnet ist, bevor der Grundkörper zusammen mit dem Hautfaden durch den Venenkatheter hindurch zurückgezogen wird.

Zumindest das Halteelement kann aus einem Material gebildet sein, welches von einem Material wenigstens eines weiteren Teilbereichs des Grundkörpers verschieden ist. Auf diese Weise lässt sich das Halteelement besonders einfach bereitstellen. Beispielsweise kann durch Verwendung eines entsprechenden Materials in dem zweiten Endbereich des Mandrins das Halteelement bereitgestellt sein.

Besonders einfach und aufwandsarm ist es bei einer nicht unter den Patentanspruch 1 fallenden Variante des Mandrins, wenn das wenigstens eine Halteelement als Haken ausgebildet ist. Durch eine hakenförmige beziehungsweise spornförmige Ausbildung des Halteelements lässt sich nämlich eine sehr einfache und zuverlässige Fixierung des Hautfadens erreichen.

Dies gilt in analoger Weise, wenn das wenigstens eine Halteelement als Öse ausgebildet ist, welche ein Einklemmen eines Teilbereichs des zu transplantierenden Hautfadens ermöglicht.

Zusätzlich oder alternativ kann bei der nicht unter den Patentanspruch 1 fallenden Variante des Mandrins das wenigstens eine Halteelement zu diesem Zweck als Schlinge ausgebildet sein. Insbesondere, wenn eine derartige Schlinge zusammenziehbar ausgebildet ist, lässt sich eine sehr sichere Fixierung zumindest eines Teilbereichs des Hautfadens während des Zurückziehens des Grundkörpers durch den Venenkatheter hindurch erreichen.

Die Schlinge kann bereitgestellt sein, indem ein Teilbereich des Grundkörpers flexibel beziehungsweise verformbar ausgebildet ist, wobei in diesem Teilbereich eine Verformung bei einem Hineinziehen der Schlinge in den Venenkatheter hinein erreichbar sein kann. Es können also insbesondere durch das Zurückziehen der Schlinge in den Venenkatheter die flexiblen Materialbereiche der Schlinge aufeinander zu bewegt werden und so das Einklemmen des Teilbereichs des Hautfadens bewirken.

Zusätzlich oder alternativ kann die Schlinge derart zusammenziehbar ausgebildet sein, dass sich bei dem Zusammenziehen der Schlinge ein von der Schlinge umschlossener Raum verkleinert. Auch dies ist für eine sichere Fixierung des Hautfadens beim Festhalten desselben vorteilhaft.

Im Hinblick auf eine Formgebung und/oder Orientierung des wenigstens einen Halteelements sind eine Vielzahl von Ausgestaltungen möglich. Beispielsweise kann das als Öse und/oder als Schlinge ausgebildete Halteelement eine rundliche aber auch eine ellipsenförmige oder eine längliche Gestalt aufweisen.

Des Weiteren kann das beispielsweise als Haken und/oder als Öse und/oder als Schlinge ausgebildete Halteelement bezogen auf einen im Wesentlichen axialen Teilbereich des Grundkörpers transversal beziehungsweise senkrecht oder schräg ausgerichtet sein. Des Weiteren ist es möglich, dass sich das beispielsweise als Haken und/oder als Öse und/oder als Schlinge ausgebildete Halteelement im Wesentlichen in die Richtung dieses axialen Teilbereichs des Grundkörpers erstreckt.

Zusätzlich oder alternativ kann das wenigstens eine Halteelement durch einen in dem Grundkörper ausgebildeten Schlitz bereitgestellt sein, welcher sich zu einem Grund des Schlitzes hin verjüngt. Auch mittels eines derartigen Schlitzes lässt sich das Festhalten des Hautfadens durch Einklemmen etwa eines Endbereichs desselben sehr einfach und zuverlässig realisieren.

Zusätzlich oder alternativ kann der in dem Grundkörper ausgebildete Schlitz einander gegenüberliegende Seitenflanken aufweisen, an welchen eine Riffelung ausgebildet ist, die ein Herausrutschen des Hautfadens aus dem Schlitz behindert. Auch auf diese Weise ist ein sehr sicheres Festhalten des Hautfadens während des Zurückziehens des Grundkörpers durch den Venenkatheter hindurch erreichbar.

Der Schlitz kann insbesondere in einem Endbereich des Grundkörpers ausgebildet sein, so dass eine vereinfachte Zugänglichkeit zu dem durch den Schlitz bereitgestellten Halteelement gegeben ist.

Gemäß einer weiteren nicht unter den Patentanspruch 1 fallenden Variante des Mandrins kann das wenigstens eine Halteelement ein aus einem elastischen Material gebildetes Schlauchstück umfassen oder durch ein solches Schlauchstück bereitgestellt sein. Durch Einbringen eines Teilbereichs, insbesondere eines Endbereichs, des Hautfadens in das elastische Schlauchstück lässt sich nämlich ebenfalls eine sichere Festlegung des Hautfadens realisieren.

In analoger Weise kann bei der nicht unter den Patentanspruch 1 fallenden Variante des Mandrins das wenigstens eine Halteelement eine Extensionshülse umfassen oder durch eine Extensionshülse bereitgestellt sein. Bei einer derartigen, aus einem Geflecht gebildeten Hülse kann durch Aufbringen einer Zugkraft erreicht werden, dass sich ein Durchmesser derselben verringert und so das Festhalten des zu transplantierenden Hautfadens erreicht wird. Auch ein derartiges Halteelement des Mandrins lässt sich technisch einfach realisieren.

Zusätzlich oder alternativ kann der Grundkörper zumindest bereichsweise aus einem Material gebildet sein, welches eine höhere Wärmeleitfähigkeit aufweist als wenigstens eine weitere Komponente des Mandrins. Hierbei ist durch ein Beaufschlagen des Materials mit Wärme und/oder durch Abführen von Wärme über das Material das Festhalten des Hautfadens mittels des wenigstens einen Halteelements bewirkbar, oder es kann das Festhalten des Hautfadens zumindest unterstützt werden.

Dem liegt die Erkenntnis zugrunde, dass durch Beaufschlagen des Materials mit Kälte ein Gewebe wie etwa das Gewebe des Hautfadens durch Anfrieren zweitweise fixiert werden kann. Dadurch lässt sich erreichen, dass beim Zurückziehen des Grundkörpers durch den Venenkatheter hindurch der zu transplantierende Hautfaden an dem die höhere Wärmeleitfähigkeit aufweisenden Material gehalten beziehungsweise fixiert ist.

Des Weiteren kann durch das Beaufschlagen des Materials mit Wärme und somit durch das Einbringen von Wärme in das Halteelement eine lokale Koagulation von Gewebebestandteilen des Gewebes des Hautfadens erreicht werden, um auf diese Weise das Festhalten des Hautfadens mittels des Halteelements zu realisieren.

Besonders einfach lässt sich das Festhalten des Hautfadens aufgrund des Beaufschlagens des Materials mit Wärme und/oder durch das Abführen von Wärme erreichen, wenn der Grundkörper zumindest bereichsweise aus einem metallischen Material gebildet ist.

Beispielsweise kann der Grundkörper bereichsweise aus dem die höhere Wärmeleitfähigkeit aufweisenden Material gebildet sein, indem eine aus einem Kunststoff gebildete Hülle des Grundkörpers einen metallischen Kern des Grundkörpers umschließt. So kann über diesen metallischen Kern sehr gut die Beaufschlagung des Hautfadens mit Wärme und dementsprechend eine Halterung durch Koagulation beziehungsweise das Abführen von Wärme von dem Hautfaden und dementsprechend eine Halterung des Hautfadens durch Festfrieren erreicht werden.

Als weiter vorteilhaft hat es sich gezeigt, wenn der Grundkörper hohl ausgebildet ist, wobei zumindest im Bereich des wenigstens einen Halteelements wenigstens eine Ansaugöffnung ausgebildet ist. Hierbei ist durch Beaufschlagen der wenigstens einen Ansaugöffnung mit einem Unterdruck ein Festhalten des Hautfadens bewirkbar oder zumindest unterstützbar. Auch eine derartige Ausgestaltung sorgt für eine zuverlässige Fixierung des Hautfadens an dem Halteelement im Bereich der wenigstens einen Ansaugöffnung.

Hierfür kann insbesondere in dem ersten Endbereich des Mandrins etwa mittels einer Spritze oder dergleichen Sauginstrument aufgrund eines Ansaugens von Luft der Unterdruck im Bereich der wenigstens einen Ansaugöffnung bereitgestellt werden. Insbesondere wenn der Unterdruck während des Zurückziehens des Grundkörpers durch den Venenkatheter hindurch aufrechterhalten wird, ist eine besonders sichere Fixierung des Hautfadens während des Hineinziehens desselben in den Venenkatheter erreichbar. Durch Lösen des Unterdrucks und/oder durch Aufbringen eines Überdrucks im Bereich der wenigstens einen Ansaugöffnung kann des Weiteren der Hautfaden besonders einfach wieder freigegeben werden. Dies erfolgt vorzugsweise, wenn der Hautfaden von dem Venenkatheter geschützt an der gewünschten Stelle im Gewebe platziert ist.

Wenn im Bereich des wenigstens einen Halteelements eine Mehrzahl von Ansaugöffnungen ausgebildet ist, so lässt sich eine besonders wirksame Halterung des Hautfadens durch Aufbringen des Unterdrucks realisieren. Insbesondere kann zusätzlich ein Einklemmen zwischen Teilstücken des Grundkörpers realisiert werden, wobei wenigstens eines der Teilstücke die wenigstens eine Ansaugöffnung aufweist.

Wenn vorgesehen ist, dass der Hautfaden zwischen den wenigstens zwei Teilstücken des hohlen Grundkörpers einklemmbar ist, so ist die wenigstens eine im Bereich des Halteelements ausgebildete Ansaugöffnung vorzugsweise an einer Seite zumindest eines der Teilstücke angeordnet oder ausgebildet, welche beim Einklemmen des Hautfadens an dem Hautfaden anliegt. So kann das Beaufschlagen der Ansaugöffnung mit dem Unterdruck in besonders wirkungsvoller Weise das Halten des Hautfadens unterstützen.

Als weiter vorteilhaft hat es sich gezeigt, wenn der Grundkörper hohl ausgebildet ist, wobei das wenigstens eine Halteelement ein durch den hohlen Grundkörper hindurchschiebbares Filament umfasst. Hierbei ist ein Teilbereich des Hautfadens aufgrund eines Zurückziehens eines aus dem hohlen Grundkörper herausstehenden Endstücks des Filaments in den Grundkörper zwischen einer Innenseite des hohlen Grundkörpers und dem Filament einklemmbar. Auch auf diese Weise lässt sich sehr einfach und zuverlässig das Festhalten des zu transplantierenden Hautfadens während des Zurückziehens des Grundkörpers durch den Venenkatheter hindurch sicherstellen.

Als weiter vorteilhaft hat es sich bei einer nicht unter den Patentanspruch 1 fallenden Variante des Mandrins gezeigt, wenn das wenigstens eine Halteelement eine Ringform aufweist. Hierbei ist zwischen einem freien Ende der Ringform und einem von dem freien Ende beabstandeten Teilbereich der Ringform eine Trennstelle ausgebildet. Dann, wenn die Ringform an einem Widerlager anliegt, ist durch eine Rotation des Grundkörpers in eine erste Richtung das freie Ende auf den Teilbereich zu bewegbar. Durch eine Rotation des Grundkörpers in eine zweite Richtung, welche der ersten Richtung entgegengesetzt ist, ist das freie Ende von dem Teilbereich weg bewegbar.

Beispielsweise kann das Halteelement nach Art einer ringförmigen Öse ausgebildet sein, wobei durch den Hautfaden das Widerlager bereitgestellt werden kann. Wenn also der Hautfaden an der Öse anliegt oder auf der Öse aufliegt, kann ein Schließen der Öse durch die Rotation des Grundkörpers beziehungsweise Drehen des Grundkörpers in die erste Richtung bewirkt werden. Auf diese Weise lässt sich eine sichere Haltung des Hautfadens mittels des Halteelements erreichen. Des Weiteren kann durch die Rotation des Grundkörpers in die erste Richtung vorzugsweise bewirkt werden, dass sich eine Steifigkeit der Ringform beziehungsweise des ringförmigen Halteelements erhöht, wobei auf diese Weise das Fixieren des Hautfadens erleichtert ist. Wenn dann der Grundkörper in die zweite Richtung gedreht wird, lässt sich der Hautfaden sehr einfach wieder freigeben, indem das die Ringform aufweisende Halteelement quasi entriegelt wird.

Zusätzlich oder alternativ ist es möglich, einen Teilbereich des Hautfadens zwischen dem freien Ende der Ringform und dem von dem freien Ende beabstandeten Teilbereich der Ringform anzuordnen und dann durch die Rotation des Grundkörpers in die erste Richtung das Einklemmen des Teilbereichs des Hautfadens zwischen dem freien Ende der Ringform und dem Teilbereich der Ringform zu bewirken. Auch dies ist im Hinblick auf ein sicheres Halten des Hautfadens mittels des Halteelements vorteilhaft.

Zusätzlich oder alternativ kann das wenigstens eine Halteelement eine Wendel umfassen, wobei nach einem Fixieren eines freien Endes der Wendel die Wendel durch eine Rotation des Grundkörpers in eine erste Richtung in eine Haltestellung verbringbar ist. In der Haltestellung ist ein Teilbereich des Hautfadens von Windungen der Wendel enger umschließbar als in einer Freigabestellung der Wendel. In die Freigabestellung ist die Wendel durch eine Rotation des Grundkörpers in eine zweite Richtung verbringbar, welcher der ersten Richtung entgegengesetzt ist. Auch durch ein derartiges, helixartiges beziehungsweise wendelförmiges Halteelement lässt sich der Teilbereich des Hautfadens sicher fixieren. Zudem lässt sich auf diese Weise auch das Freigeben des Teilbereichs des Hautfadens sehr einfach bewerkstelligen, nämlich durch die Rotation des Grundkörpers in die zweite Richtung.

Um das Fixieren des Teilbereichs des Hautfadens in einem von den Windungen der Wendel umgebenen Aufnahmeraum weiter zu verbessern, kann die Wendel eine Mehrzahl von Widerhaken aufweisen, welche ein Herausrutschen des Teilbereichs aus dem von den Windungen der Wendel beziehungsweise des helixförmigen Halteelements umschlossenen Bereich oder Aufnahmeraum heraus verhindern.

Die vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Es sind somit auch Ausführungen als von der Erfindung umfasst und offenbart anzusehen, die in den Figuren nicht explizit gezeigt oder erläutert sind, jedoch durch separierte Merkmalskombinationen aus den erläuterten Ausführungen hervorgehen und erzeugbar sind. Es sind somit auch Ausführungen und Merkmalskombinationen als offenbart anzusehen, die nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen.

Weitere Vorteile, Merkmale und Einzelheiten der Erfindung ergeben sich aus den Ansprüchen, der nachfolgenden Beschreibung bevorzugter Ausführungsformen sowie anhand der Zeichnungen. Dabei zeigen:
- Fig. 1: schematisch einen Mandrin mit einem eigensteifen Grundkörper, welcher durch einen peripheren Venenkatheter hindurchgeführt ist und an seinem freien beziehungsweise distalen Ende ein Halteelement zum Halten eines korialen Hautfadens aufweist;
- Fig. 2: stark schematisiert und teilweise den Grundkörper gemäß Fig. 1 mit einer Variante des Halteelements;
- Fig. 3: schematisch eine weitere Variante des Mandrins, wobei der Grundkörper des Mandrins über seine gesamte Länge in zwei voneinander separate Teilstück getrennt ist;
- Fig. 4: schematisch eine weitere Variante des Mandrins, bei welcher das Halteelement als verdrillbare Öse ausgebildet ist;
- Fig. 5: die Variante gemäß Fig. 4 in einer Entriegelungsstellung;
- Fig. 6: eine weitere Variante des Mandrins, bei welcher das Halteelement nach Art einer länglichen Schlinge oder Öse ausgebildet ist;
- Fig. 7: schematisch mögliche Ausrichtungen des Halteelements relativ zu einem axialen Abschnitt des Grundkörpers des Mandrins;
- Fig. 8: schematisch eine weitere Variante des Mandrins, bei welcher das Halteelement nach Art einer Wendel ausgebildet ist;
- Fig. 9: eine weitere Variante des Mandrins, bei welcher das Halteelement nach Art eines Hakens ausgebildet ist;
- Fig. 10: eine weitere Variante des Mandrins, bei welcher in einem Endbereich des Grundkörpers ein Schlitz ausgebildet ist, welcher zum Einklemmen des Hautfadens geeignet ist;
- Fig. 11: eine weitere Variante des Mandrins, bei welcher das Halteelement durch einen sich V-förmig verjüngenden Schlitz gebildet ist; und
- Fig. 12: eine weitere Variante des Mandrins, bei welcher der Grundkörper hohl ausgebildet ist und das Halteelement ein durch den hohlen Grundkörper hindurchschiebbares Filament umfasst.

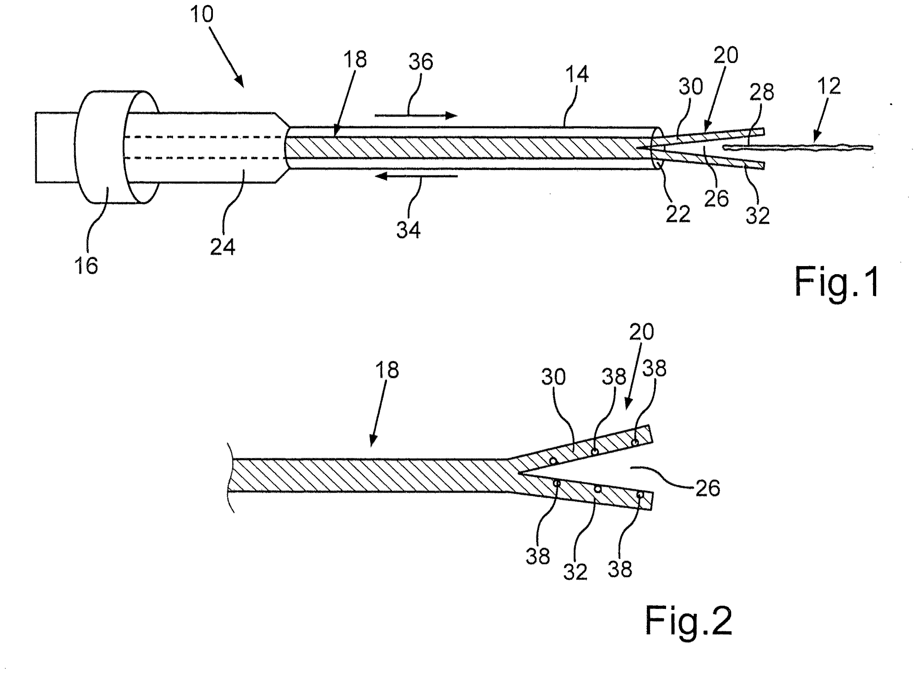

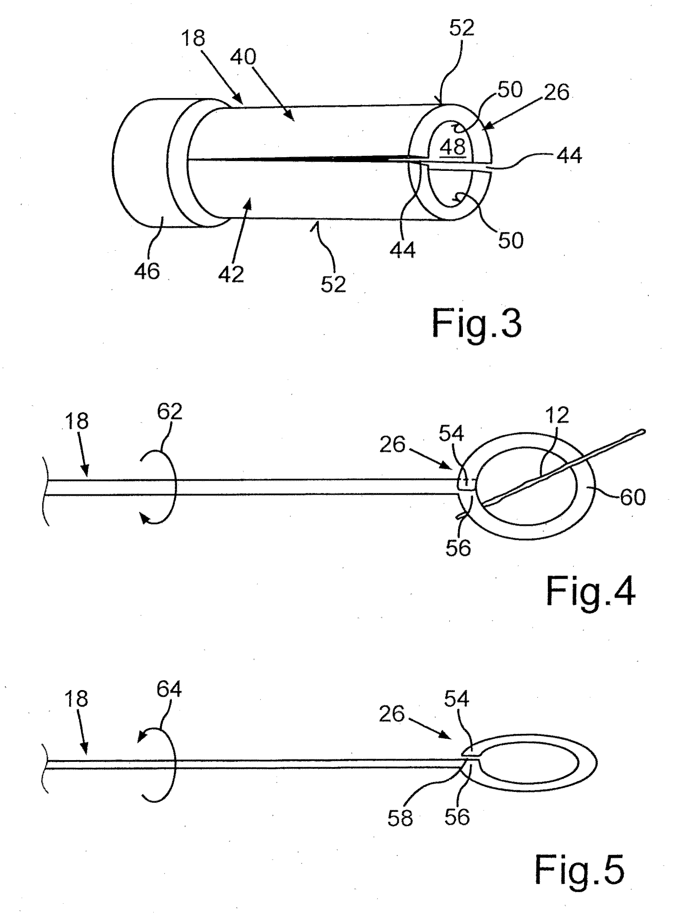

In den Figuren, in denen Fig. 1 einer Erläuterung des Hintergrunds der Erfindung dient, und in denen Fig. 4 bis Fig. 12 in Alleinstellung keine eigenständigen Ausführungsbeispiele der im Patentanspruch 1 beanspruchten Erfindung darstellen, sind gleiche oder funktionsgleiche Elemente mit identischen Bezugszeichen versehen.

In Fig. 1 ist stark schematisiert ein Mandrin 10 gezeigt, welcher zum Transplantieren eines Hautfadens 12 geeignet ist. Bei dem Hautfaden 12 oder dergleichen Transplantat aus menschlichem Gewebe kann es sich insbesondere um einen korialen Hautfaden 12 handeln, also um einen Hautfaden 12, welcher aus der menschlichen Lederhaut gewonnen wurde. Insbesondere kann vorgesehen sein, einen derartigen Hautfaden 12 im Bereich eines Gewebedefekts beziehungsweise Hautdefekts in die im Bereich des Gewebedefekts vorhandene Lederhaut einzubringen. Hierfür wird in der (vorliegend nicht gesondert dargestellten) straffen Lederhaut zunächst eine Hülle etwa in Form eines in Fig. 1 beispielhaft gezeigten Venenkatheters 14 angeordnet. Der Venenkatheter 14 ist hierbei vorzugsweise als peripherer Venenkatheter oder Venenverweilkatheter mit einem entsprechenden, für derartige Venenkatheter 14 gängigen Durchmesser und einer gängigen Länge ausgebildet. Durch den vorliegend beispielhaft gezeigten Venenkatheter 14 ist in der Lederhaut somit eine Schutzhülle bereitgestellt, innerhalb welcher der Hautfaden 12 platziert werden kann.

Zum Einbringen des Venenkatheters 14 in die Lederhaut kann die Lederhaut zunächst mit einer innerhalb des Venenkatheters 14 angeordneten (vorliegend nicht gezeigten) Stahlkanüle beziehungsweise Stahlnadel durchstochen werden, welche an einem distalen oder freien Ende 22 des Venenkatheters 14 aus dem Venenkatheter 14 ein Stück weit heraussteht. In dem auf diese Weise in der Lederhaut gebildeten oder geformten Tunnel wird dann der Venenkatheter 14 angeordnet. Nach dem Zurückziehen der Stahlkanüle beziehungsweise Stahlnadel hält der Venenkatheter 14 in der Lederhaut einen Bereich offen, in welchen der Hautfaden 12 eingebracht werden kann.

Für ein derartiges Transplantieren des Hautfadens 12 etwa in die menschliche Lederhaut kann der in Fig. 1 schematisch dargestellte Mandrin 10 verwendet werden. Der Mandrin 10 weist einen ersten Endbereich auf, welcher eine Mandrinkappe 16 umfassen kann. Insbesondere im Bereich dieser Mandrinkappe 16 lässt sich der Mandrin 10 greifen, wenn ein eigensteifer Grundkörper 18 des Mandrins 10 durch den Venenkatheter 14 hindurchgeschoben werden soll.

Der entsprechende Zustand, in welchem ein freier Endbereich 20 beziehungsweise ein distales Ende des Grundkörpers 18 und somit auch des Mandrins 10 an dem distalen oder freien Ende 22 des Venenkatheters 14 aus dem Venenkatheter 14 heraustritt, ist in Fig. 1 schematisch gezeigt. Eine sich an die Mandrinkappe 16 anschließende Manschette 24, innerhalb welcher bei dem in Fig. 1 beispielhaft gezeigten Mandrin 10 der eigensteife Grundkörper 18 angeordnet ist, kann in dieser Konstellation an ein gegenüberliegendes, proximales Ende des Venenkatheters 14 anstoßen.

Durch den beispielsweise die Mandrinkappe 16 und die Manschette 24 umfassenden ersten Endbereich des Mandrins 10 ist somit bei der in Fig. 1 gezeigten Variante des Mandrins 10 ein Anschlag für das Hindurchschieben des Grundkörpers 18 durch den Venenkatheter 14 bereitgestellt. Mit anderen Worten sorgt der durch den ersten Endbereich des Mandrins 10 gebildete Anschlag dafür, dass eine Bewegung des Grundkörpers 18 in eine Einschubrichtung 36 begrenzt ist, wenn der Grundkörper 18 durch den Venenkatheter 14 hindurchgeschoben wird. Die Einschubrichtung 36 ist in Fig. 1 durch einen Pfeil veranschaulicht.

Ob der durch den ersten Endbereich des Mandrins 10 gebildete Anschlag tatsächlich wie in Fig. 1 gezeigt an dem proximalen Ende des Venenkatheters 14 anstößt, ist jedoch von der Länge des Mandrins 10 und des Grundkörpers 18 relativ zu der Länge des Venenkatheters 14 abhängig. Wenn also der Grundkörper 18 länger ist als vorliegend beispielhaft gezeigt, so kann zwischen dem proximalen Ende des Venenkatheters 14 und der Manschette 24 ein Abstand vorliegen.

Wie in Fig. 1 schematisch gezeigt, kann die den Grundkörper 18 umgebende Manschette 24 aus einem transparenten Material, insbesondere aus einem Kunststoffmaterial, gebildet sein.

Es ist auch möglich, dass der erste Endbereich des Mandrins 10 durch einen dem freien Endbereich 20 gegenüberliegenden weiteren Endbereich des Grundkörpers 18 bereitgestellt ist. Jedoch lässt sich der Mandrin 10 besonders gut handhaben, wenn der Mandrin 10 die Mandrinkappe 16 aufweist. Insbesondere zu diesem Zweck können in Varianten des Mandrins 10 auch lediglich die Mandrinkappe 16 oder lediglich die Manschette 24 vorgesehen sein.

Bei der in Fig. 1 gezeigten Ausgestaltung des Mandrins 10 weist der Mandrin 10 in dem zweiten Endbereich 20 ein Halteelement 26 auf, welches dazu ausgebildet ist, während eines Zurückziehens des Grundkörpers 18 durch den Venenkatheter 14 hindurch den zu transplantierenden Hautfaden 12 in einem Endbereich 28 des Hautfadens 12 festzuhalten.

Bei der in Fig. 1 beispielhaft gezeigten Ausgestaltung ist das Halteelement 26 nach Art einer zwei Arme 30, 32 aufweisenden Pinzette ausgebildet. Mit anderen Worten ist bei dieser Ausgestaltung des Halteelements 26 der Grundkörper 18 in dem zweiten Endbereich 20 in zwei Teilstücke in Form der Arme 30, 32 gespalten. Zwischen diesen Armen 30, 32 oder Teilstücken kann der Endbereich 28 des Hautfadens 12 angeordnet und eingeklemmt werden. Insbesondere kann dieses Einklemmen des Endbereichs 28 des Hautfadens 12 erreicht werden, indem der Grundkörper 18 in eine Richtung 34 durch den Venenkatheter 14 hindurchgezogen wird, welche in Fig. 1 durch einen Pfeil veranschaulicht ist.

Diese Richtung 34, in welche der Mandrin 10 relativ zu dem Venenkatheter 14 bewegt werden kann, während der Grundkörper 18 durch den Venenkatheter 14 hindurchgezogen wird, ist der Einschubrichtung 36 entgegengesetzt. In die Einschubrichtung 36 wird der Grundkörper 18 des Mandrins 10 durch den Venenkatheter 14 hindurchgeschoben, so dass der freie, das Halteelement 26 aufweisende Endbereich 20 des Mandrins 10 über das freie oder distale Ende 22 des Venenkatheters 14 übersteht und so mittels des Halteelements 26 der Hautfaden 12 gepackt oder gegriffen werden kann.

insbesondere werden die pinzettenartigen Arme 30, 32 des Grundkörpers 18 aufeinander zu bewegt, wenn der freie Endbereich 20 des Grundkörpers 18 in den Venenkatheter 14 an dessen freiem oder distalem Ende 22 eintritt. Somit kann durch das Zurückziehen des Grundkörpers 18 durch den Venenkatheter 14 hindurch das Einklemmen des Endbereichs 28 des Hautfadens 12 besonders einfach und zuverlässig bewirkt werden.

Wenn der Hautfaden 12 anschließend in den Venenkatheter 14 hineingezogen ist, kann der Endbereich 28 des Hautfadens 12 wieder aus dem Halteelement 26 freigegeben werden. Dann ist der Hautfaden 12 nämlich durch den Venenkatheter 14 geschützt in dem Gewebe platziert, in welchem der Hautfaden 12 angeordnet werden sollte, beispielsweise also in der Lederhaut beziehungsweise dem Korium der Haut, welche einen aufzufüllenden oder auszugleichenden Gewebedefekt aufweist.

Es kann dann der Hautfaden 12 beispielsweise an seinem dem Endbereich 28 gegenüberliegenden Ende festgehalten werden, während der Venenkatheter 14 in die in Fig. 1 durch den Pfeil veranschaulichte Richtung 34 aus der Lederhaut herausgezogen wird. Dadurch kommt der beispielsweise koriale Hautfaden 12 in dem straffen Korium zu liegen und kann dort als körpereigenes Gewebe in einem gut durchbluteten und eng anliegenden Wundbett als ganzes, nicht fraktioniertes Transplantat einheilen. Eine unerwünschte Verschiebung des in das Korium eingebrachten Hautfadens 12 ist aufgrund der vorstehend geschilderten Verwendung des Mandrins 10 und des Venenkatheters 14 besonders weitgehend vermieden.

In Fig. 2 ist stark schematisiert das in dem Endbereich 20 des Mandrins 10 beziehungsweise des Grundkörpers 18 ausgebildete Halteelement 26 gemäß einer Variante gezeigt. Hierbei ist der Grundkörper 18 hohl ausgebildet, und im Bereich der Arme 30, 32 ist eine Mehrzahl von Ansaugöffnungen 38 ausgebildet, von welchen aus Gründen der Übersichtlichkeit in Fig. 2 lediglich einige mit Bezugszeichen versehen sind. Durch Anlegen eines Unterdrucks im Bereich der Mandrinkappe 16, beispielsweise mittels einer in die Mandrinkappe 16 des Mandrins 10 eingeschobenen Spritze, kann auch im Bereich der Ansaugöffnungen 38 ein Unterdruck aufgebracht werden. Dieser Unterdruck kann das Halten des Endbereichs 28 des Hautfadens 12 (vergleiche Fig. 1) mittels des Halteelements 26 bewirken oder das Festhalten zumindest unterstützen.

Bei den in Fig. 1 und Fig. 2 gezeigten Varianten des Mandrins 10 ist der Grundkörper 18 lediglich in dem zweiten Endbereich 20 in die zwei Teilstücke etwa in Form der Arme 30, 32 gespalten oder geteilt. Es kann jedoch auch vorgesehen sein, dass der Grundkörper 18 in dem Endbereich 20 in mehr als die vorliegend beispielhaft gezeigten zwei Teilstücke beziehungsweise Arme 30, 32 gespalten oder geteilt ist. Des Weiteren ist in Fig. 3 stark schematisiert eine Ausgestaltung des Mandrins 10 gezeigt, bei welcher der Grundkörper 18 über seine gesamte Länge in zwei Teilstücke 40, 42 geteilt ist.

Entsprechende, sich in eine Längsrichtung oder axiale Richtung des Grundkörpers 18 erstreckende Trennstellen 44 sind in Fig. 3 schematisch gezeigt. Aufgrund der Trennung des Grundkörpers 18 in die wenigstens zwei Teilstücke 40, 42 können nach dem Verbringen des Hautfadens 12 in seine Zielposition die beiden Teilstücke 40, 42 sehr einfach separat voneinander beziehungsweise nacheinander entfernt werden. Dies ist insbesondere dann vorteilhaft, wenn sich bei diesem Entfernen oder Entnehmen ein Endbereich des jeweiligen Teilstücks 40, 42 noch innerhalb des (in Fig. 3 nicht gezeigten) Venenkatheters 14 befindet.

Um einen Zusammenhalt der voneinander separaten Teilstücke 40, 42 zu erreichen, weist bei der in Fig. 3 gezeigten Ausgestaltung des Mandrins 10 der Mandrin 10 ein beispielsweise nach Art einer Hülse 46 ausgebildetes Kopplungselement auf. Die Hülse 46 kann beispielsweise aus Gummi oder einem derartigen elastischen Material gebildet sein und insbesondere in dem ersten Endbereich des Mandrins 10 die beiden Teilstücke 40, 42 zusammenhalten.

Entsprechend kann bei dieser Ausgestaltung auch anstelle der Mandrinkappe 16 (vergleiche Fig. 1) die Hülse 46 vorgesehen sein. Bei dieser Ausgestaltung des Mandrins 10 kann insbesondere durch die Hülse 46 der Anschlag für das Hindurchschieben des Grundkörpers 18 durch den Venenkatheter 14 hindurch bereitgestellt oder gebildet sein. Es ist jedoch auch möglich, die Hülse 46 zusätzlich zu der Mandrinkappe 16 vorzusehen.

Aus der schematischen Darstellung in Fig. 3 ist besonders gut erkennbar, dass die beiden Teilstücke 40, 42 nach Art von im Querschnitt halbkreisbogenförmigen Teilschalen ausgebildet sein können. Eine derartige Ausgestaltung ist auch bei den in Fig. 1 und Fig. 2 gezeigten Teilstücke oder Armen 30, 32 möglich. Dementsprechend begrenzen die Teilstücke 40, 42 einen Aufnahmeraum 48, in welchen der Endbereich 28 des Hautfadens 12 eingebracht werden kann, um diesen Endbereich 28 zu fixieren.

Anhand von Fig. 3 kann weiter besonders gut veranschaulicht werden, dass die beiden Teilstücke 40, 42 ebenso wie die in Fig. 1 und Fig. 2 schematisch gezeigten Arme 30, 32 jeweils eine dem zu haltenden Endbereich 28 des Hautfadens 12 zugewandte Halteseite 50 und eine der Halteseite 50 gegenüberliegende oder abgewandte Außenseite 52 aufweisen. Hierbei kann eine Rauheit der Halteseite 50 größer sein als eine Rauheit der Außenseite 52. Auf diese Weise kann erreicht werden, dass der Grundkörper 18 besonders reibungsarm durch den Venenkatheter 14 hindurchgezogen werden kann. Demgegenüber bewirkt die im Verhältnis größere Rauheit der Halteseite 50 ein gutes Fixieren des Hautfadens 12, insbesondere in dessen Endbereich 28 (vergleiche Fig. 1).

Die Halteseiten 50 der beiden Teilstücke 40, 42 brauchen nicht wie in Fig. 3 schematisch gezeigt konkav gewölbt oder gekehlt ausgebildet zu sein. Es ist vielmehr ebenso möglich, das Halteelement 26 durch die wenigstens zwei voneinander separaten Teilstücke 40, 42 oder durch die wenigstens zwei Arme 30, 32 (vergleiche Fig. 1 und Fig. 2) bereitzustellen, wenn die dem Hautfaden 12 zugewandten Halteseiten 50 (oder zumindest eine dieser Halteseiten 50) eben beziehungsweise gerade ausgebildet sind. Bei einer runden Außenkontur des Grundkörpers 18 können demnach die beiden Teilstücke 40, 42 oder die beiden Arme 30, 32 insbesondere im Querschnitt die Form eines jeweiligen Halbkreises aufweisen.

Bei der in Fig. 4 gezeigten Variante des Mandrins 10 ist das Halteelement 26 nach Art einer Öse ausgebildet. Hierbei weist das Halteelement 26 eine Ringform auf. Zwischen einem freien Ende 54 der Ringform und einem weiteren Teilbereich 56 der Ringform ist eine Trennstelle 58 ausgebildet, welche in Fig. 5 besser erkennbar ist als in Fig. 4. Denn in Fig. 4 ist das die Ringform aufweisende Halteelement 26 in einer Schließstellung gezeigt, in welcher der Hautfaden 12 im Bereich der ringförmigen Öse gehalten beziehungsweise fixiert ist. Der Hautfaden 12 kann hierbei durch die ringförmige Öse hindurchgefädelt sein, so dass der Hautfaden 12 in dem Teilbereich 56, welcher unterhalb des freien Endes 54 angeordnet ist beziehungsweise dem freien Ende 54 gegenüberliegt, oder auch an einer anderen Stelle unterhalb der Ringform verläuft.

Wie in Fig. 4 gezeigt, kann in einem anderen Teilbereich 60 des ringförmigen Halteelements 26 der Hautfaden 12 auf dieser ringförmigen Öse aufliegen. Insbesondere aufgrund einer derartigen Anordnung des durch die Öse hindurchgefädelten Hautfadens 12 kann durch den Hautfaden 12 ein Widerlager bereitgestellt sein. Wird der Grundkörper 18 etwa an seinem proximalen Ende in eine erste Richtung gedreht, welche in Fig. 4 durch einen gekrümmten Pfeil 62 veranschaulicht ist, so bewirkt dies ein Schließen der ringförmigen Öse beziehungsweise des die Ringform aufweisenden Halteelements 26 und hierbei ein Fixieren des Hautfadens 12, insbesondere durch Einklemmen des Hautfadens 12.

Letzteres kann insbesondere erreicht werden, indem ein Stück des Hautfadens 12 in dem Bereich der Trennstelle 58 angeordnet wird, bevor der Grundkörper 18 in die Drehrichtung gedreht wird, welche in Fig. 4 durch den gekrümmten Pfeil 62 veranschaulicht ist. Durch das Verbringen der Öse in die Schließstellung weist die Öse zudem eine erhöhte Steifigkeit beziehungsweise Festigkeit auf, welche im Hinblick auf das Festhalten des Hautfadens 12 vorteilhaft ist.

Demgegenüber kann zum Freigeben des Hautfadens 12 der Grundkörper 18 anschließend in eine zweite Richtung gedreht werden, welche in Fig. 5 durch einen weiteren gekrümmten Pfeil 64 veranschaulicht ist. Das Verdrehen der ringförmigen Öse in diese zweite Richtung bewirkt, dass das freie Ende 54 von dem Teilbereich 56 weg bewegt wird und somit an der Trennstelle 58 ein entsprechender Spalt ausgebildet ist. In dieser Offenstellung ist die ringförmige Öse quasi entriegelt, so dass der Hautfaden 12 leicht freigegeben werden kann.

In Fig. 4 und Fig. 5 ist die ringförmige Öse beziehungsweise das die Ringform aufweisende Halteelement 26 des Mandrins 10 lediglich die Haltefunktion der Öse veranschaulichend dargestellt, nicht jedoch im Hinblick auf die tatsächlichen Abmessungen der Öse oder dergleichen.

Denn der Grundkörper 18 mitsamt dem ringförmigen beziehungsweise die Ringform aufweisenden Halteelement 26 ist in die Einschubrichtung 36 (vergleiche Fig. 1) durch den Venenkatheter 14 hindurchführbar, um nach einem Herausschieben zumindest des ringförmigen Halteelements 26 aus dem Venenkatheter 14 heraus (vergleiche Fig. 1) das Festlegen des Hautfadens 12 im Bereich des Halteelements 26 zu ermöglichen. Dies gilt in analoger Weise für sämtliche vorliegend in den Figuren schematisch gezeigten Varianten des Mandrins 10 mit dem wenigstens einen Halteelement 26.

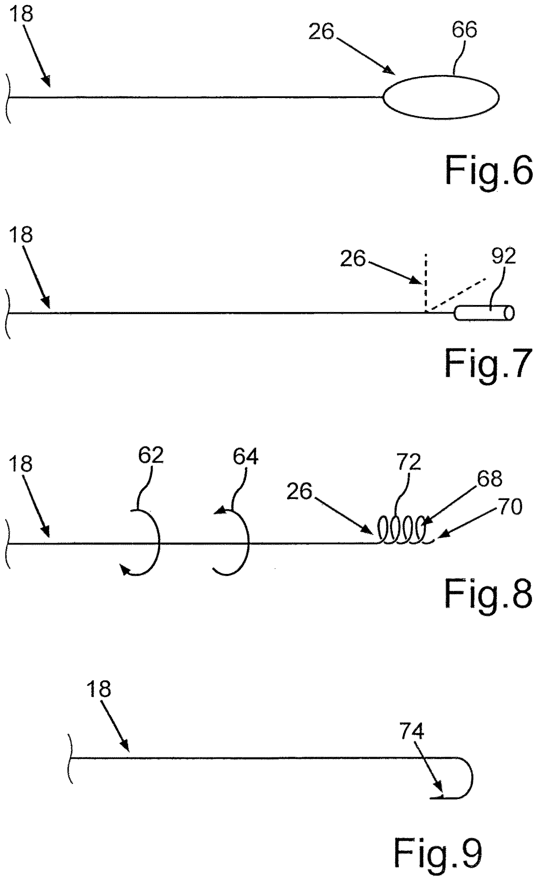

In Fig. 6 ist stark schematisiert eine weitere Variante des Mandrins 10 gezeigt, bei welchem das Halteelement 26 durch eine Schlinge 66 bereitgestellt ist, welche vorzugsweise zusammenziehbar ausgebildet ist. Die Schlinge 66 kann eine runde oder, wie in Fig. 6 schematisch angedeutet, eine längliche Gestalt aufweisen. Durch Aufbringen eines Zugs an dem Grundkörper 18 in die in Fig. 1 angegebene Richtung 34 kann vorzugsweise das Zusammenziehen der Schlinge 66 bewirkt werden.

Anhand von Fig. 7 lässt sich verdeutlichen, dass das Halteelement 26, welches vorzugsweise in dem zweiten Endbereich 20 des Grundkörpers 18 ausgebildet ist, (vergleiche Fig. 1) unterschiedliche Orientierungen relativ zu einem sich in die Einschubrichtung 36 erstreckenden Hauptbereich des Grundkörpers 18 aufweisen kann. Dementsprechend braucht sich das Halteelement 26 nicht streng in diese axiale Richtung zu erstrecken, sondern es kann auch schräg oder senkrecht zu dieser axialen Richtung ausgerichtet sein.

Aus Fig. 7 ist außerdem ersichtlich, dass das Halteelement 26 durch ein aus einem elastischen Material gebildetes Schlauchstück 92 bereitgestellt sein kann. In dieses elastische Schlauchstück 92 kann der Endbereich 28 des Hautfadens 12 eingebracht werden, um den Endbereich 28 des Hautfadens 12 (vergleiche Fig. 1) einzuklemmen. In analoger Weise kann der Mandrin 10 anstelle eines derartigen Schlauchstücks 92 oder zusätzlich zu diesem eine Extensionshülse aufweisen, durch welche dann das wenigstens eine Halteelement 26 bereitgestellt ist.

Gemäß Fig. 8 kann das Halteelement 26 nach Art einer helixförmigen beziehungsweise korkenzieherförmigen Wendel 68 ausgebildet sein beziehungsweise durch eine derartige Wendel 68 bereitgestellt sein. Wenn ein freies Ende 70 der Wendel 68 festgehalten oder fixiert wird, etwa durch Einbringen beziehungsweise Einschieben des Endbereichs 28 des Hautfadens 12 in einen Aufnahmeraum der Wendel 68 derart, dass Windungen 72 der Wendel 68 um diesen Aufnahmeraum umlaufen, so kann so wie mit Bezug auf Fig. 4 und Fig. 5 erläutert durch Rotation des Grundkörpers 18 auf einfache Weise ein Fixieren des Hautfadens 12 erreicht werden.

Wenn nämlich der Grundkörper 18 in die in Fig. 8 durch den gekrümmten Pfeil 62 veranschaulichte Drehrichtung beziehungsweise erste Richtung gedreht wird, so umschließen die Windungen 72 der verformbaren Wendel 68 den Endbereich 28 des Hautfadens 12 eng. Wird demgegenüber der Grundkörper 18 in die entgegengesetzte Drehrichtung gedreht, welche in Fig. 8 durch einen weiteren gekrümmten Pfeil 64 veranschaulicht ist, so wird die Wendel 68 in eine Freigabestellung verbracht, in welcher der Endbereich 28 des Hautfadens 12 leicht aus dem durch die Wendel 68 gebildeten Halteelement 26 entnehmbar ist.

Bei der in Fig. 9 gezeigten Variante des Mandrins 10 ist das Halteelement 26 durch einen Haken 74 bereitgestellt, welcher in dem zweiten Endbereich 20 (vergleiche Fig. 1) des Grundkörpers 18 ausgebildet sein kann. Auch mittels eines derartigen Hakens 74 lassen sich der Hautfaden 12 und insbesondere der Endbereich 28 des Hautfadens 12 gut fixieren, während der Grundkörper 18 in die in Fig. 1 gezeigte Richtung 34 durch den Venenkatheter 14 hindurchgezogen wird.

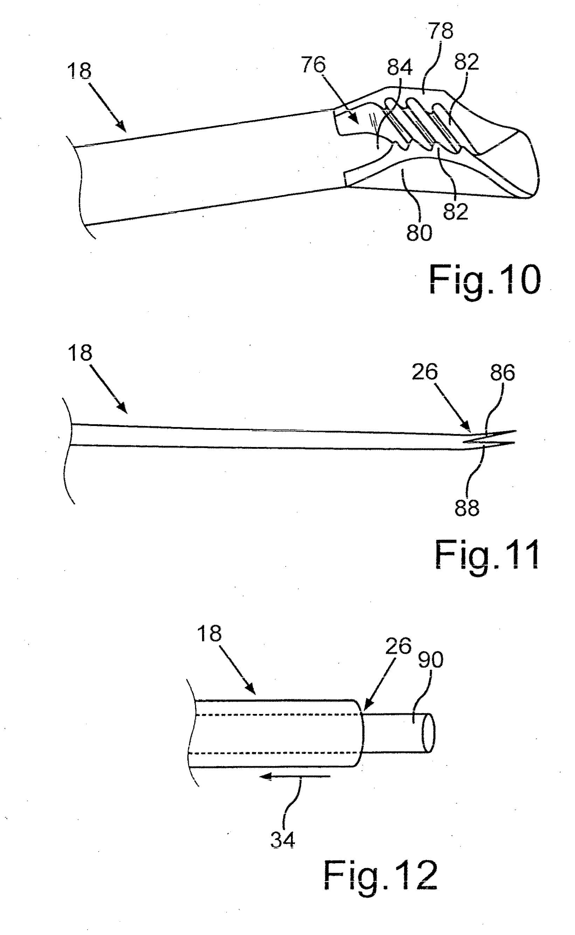

In Fig. 10 ist schematisch eine weitere Variante des Mandrins 10 ausschnittsweise gezeigt, bei welcher in dem freien Endbereich 20 des Grundkörpers 18 ein Schlitz 76 ausgebildet ist, durch welchen das Halteelement 26 bereitgestellt ist. Hierbei weist der Schlitz 76 eine erste Seitenflanke 78 und eine zweite Seitenflanke 80 auf. Die zweite Seitenflanke 80 liegt der ersten Seitenflanke 78 gegenüber. An den einander zugewandten Seitenflächen der Seitenflanken 78, 80 sind Rippen 82 ausgebildet, welche in Fig. 10 schematisch gezeigt sind.

Die durch die Rippen 82 gebildete Riffelung der Seitenflanken 78, 80 behindert oder verhindert ein Herausrutschen des (in Fig. 10 nicht gezeigten) Hautfadens 12 aus dem Schlitz 76 in die Einschubrichtung 36 (vergleiche Fig. 1). Hierfür können die Rippen 82 insbesondere, wie in Fig. 10 schematisch gezeigt, geneigt zu der Einschubrichtung 36 ausgerichtet sein. Zusätzlich oder alternativ kann vorgesehen sein, dass sich der Schlitz 76 zu einem Grund 84 hin verjüngt. Auch durch eine derartige, im Querschnitt im Wesentlichen V-förmige Ausgestaltung des Schlitzes 76 lässt sich ein gutes Einklemmen insbesondere des Endbereichs 28 des Hautfadens 12 erreichen.

In Fig. 11 ist schematisch eine weitere, besonders einfache Ausgestaltung des Halteelements 26 gezeigt, welches in dem Endbereich 20 des Grundkörpers 18 (vergleiche Fig. 1) durch einen sich V-förmig verjüngenden Schlitz bereitgestellt ist, der in dem Grundkörper 18 ausgebildet ist. Zwischen jeweiligen Schenkeln 86, 88 dieses Schlitzes lässt sich der Endbereich 28 des Hautfadens 12 (vergleiche Fig. 1) ebenfalls gut einklemmen und somit festhalten.

Bei der in Fig. 12 gezeigten Ausgestaltung des Mandrins 10 ist der Grundkörper 18 hohl ausgebildet, und durch den hohlen Grundkörper 18 kann ein etwa nach Art eines Drahts 90 der dergleichen ausgebildetes Filament des Mandrins 10 hindurchgeschoben werden. Im Zusammenwirken mit dem hohlen Grundkörper 18 ist hierbei das Halteelement 26 bereitgestellt. Wenn nämlich, wie in Fig. 12 gezeigt, ein Endstück des Filaments oder Drahts 90 aus dem freien Ende des hohlen Grundkörpers 18 heraussteht, so kann anschließend der zweite Endbereich 28 des Hautfadens 12 (vergleiche Fig. 1) zwischen einer Innenseite des hohlen Grundkörpers 18 und dem Filament beziehungsweise Draht 90 eingeklemmt werden. Hierfür kann der Draht 90 in die Richtung 34 in den hohlen Grundkörper 18 zurückgezogen werden, welche in Fig. 12 (so wie in Fig. 1) durch einen Pfeil veranschaulicht ist.

Um ein Festhalten des Endbereichs 28 des Hautfadens 12 (vergleiche Fig. 1) an dem Endstück des Drahts 90 zu erleichtern, kann der Draht 90 einen Haken, eine Öse oder dergleichen aufweisen, wie dies mit Bezug auf die weiteren Ausgestaltungen des Mandrins 10 bereits erläutert wurde.

Zusätzlich oder alternativ kann vorgesehen sein, dass durch Beaufschlagen des beispielsweise aus einem Metall gebildeten Drahts 90 mit Kälte ein Festfrieren des Hautfadens 12 an dem Draht 90 und/oder durch Beaufschlagen des Drahts 90 mit Wärme eine Koagulation von Komponenten des Hautfadens 12 und somit ein Anhaften des Hautfadens 12 an dem Draht 90 bewirkbar ist. Anschließend kann der Draht 90 in den hohlen Grundkörper 18 zurückgezogen werde, wobei der Hautfaden 12 zwischen der Innenseite des hohlen Grundkörpers 18 und dem Draht 90 eingeklemmt wird.

Ein derartiges Festhalten des Hautfadens 12 an dem Grundkörper 18 durch Festfrieren oder durch Beaufschlagen mit Wärme lässt sich auch realisieren, wenn der Grundkörper 18 nicht wie in Fig. 12 gezeigt hohl aber aus einem entsprechend gut Wärme leitenden Material gebildet ist.

## Patentansprüche

1. Mandrin zum Transplantieren eines Hautfadens (12), wobei ein Grundkörper (18) des Mandrins (10) eine Eigensteifigkeit aufweist, welche ein Hindurchschieben des Grundkörpers (18) durch einen peripheren Venenkatheter (14) zulässt, wobei der Mandrin (10) einen ersten Endbereich (16, 24) aufweist, in welchem der Mandrin (10) bei einem Zurückziehen des Mandrins (10) greifbar ist, wobei durch den ersten Endbereich (16, 24) ein Anschlag für das Hindurchschieben des Grundkörpers (18) durch den Venenkatheter (14) bereitgestellt ist, wobei durch das Zurückziehen des Mandrins (10) eine Bewegung des Grundkörpers (18) durch den Venenkatheter (14) hindurch in eine Richtung (34) bewirkbar ist, welche einer Richtung (36) bei dem Hindurchschieben entgegengesetzt ist, und wobei der Mandrin (10) zumindest in einem zweiten Endbereich (20) wenigstens ein Halteelement (26) aufweist, welches dazu ausgebildet ist, während des Zurückziehens des Grundkörpers (18) durch den Venenkatheter (14) hindurch den zu transplantierenden Hautfaden (12) festzuhalten,
wobei das Halteelement (26) wenigstens zwei Teilstücke (40, 42) des Grundkörpers (18) umfasst, welche zumindest in dem zweiten Endbereich (20) voneinander weg bewegbar sind, wobei ein zwischen den wenigstens zwei Teilstücken (40, 42) anordenbarer Teilbereich (28) des Hautfadens (12) aufgrund des Zurückziehens des Grundkörpers (18) durch den Venenkatheter (14) hindurch einklemmbar ist,
**dadurch gekennzeichnet, dass**
der Grundkörper (18) über seine gesamte Länge in die wenigstens zwei Teilstücke (40, 42) getrennt ist, welche voneinander separat ausgebildet und zum Festhalten des Hautfadens (12) voneinander weg bewegbar sind, wobei die wenigstens zwei voneinander separaten Teilstücke (40, 42) mittels eines von dem Grundkörper (18) entfernbaren Kopplungselements (46) des Mandrins (10) zusammengehalten sind.

2. Mandrin nach Anspruch 1,
**dadurch gekennzeichnet, dass**
- die wenigstens zwei voneinander separaten Teilstücke (40, 42) in dem ersten Endbereich (16, 24) mittels des Kopplungselements (46) des Mandrins (10) zusammengehalten sind.

3. Mandrin nach Anspruch 1 oder 2,
**dadurch gekennzeichnet, dass**
die wenigstens zwei Teilstücke (40, 42) in einer Haltestellung, in welcher das Einklemmen des Teilbereichs (28) des Hautfadens (12) mittels des Halteelements (26) bewirkbar ist, einen in dem Grundkörper (18) ausgebildeten Aufnahmeraum (48) begrenzen und/oder die wenigstens zwei Teilstücke (40, 42) in der Haltestellung jeweils eine dem Teilbereich (28) des Hautfadens (12) zugewandte Halteseite (50) und eine der Halteseite (50) abgewandte Außenseite (52) aufweisen, wobei eine Rauheit der Halteseite (50) größer oder gleich einer Rauheit der Außenseite (52) ist.

4. Mandrin nach einem der Ansprüche 1 bis 3,
**dadurch gekennzeichnet, dass**
der, insbesondere zu dem zweiten Endbereich (20) hin sich verjüngend ausgebildete, Grundkörper (18) zumindest im Bereich des Halteelements (26) transparent ausgebildet ist und/oder zumindest das Halteelement (26) aus einem Material gebildet ist, welches von einem Material wenigstens eines weiteren Teilbereichs des Grundkörpers (18) verschieden ist.

5. Mandrin nach einem der Ansprüche 1 bis 4,
**dadurch gekennzeichnet, dass**
das wenigstens eine Halteelement (26) durch einen in dem Grundkörper (18), insbesondere in einem Endbereich (20) des Grundkörpers (18), ausgebildeten Schlitz (76) bereitgestellt ist, welcher sich zu einem Grund (84) des Schlitzes (76) hin verjüngt und/oder welcher einander gegenüberliegende Seitenflanken (78, 80) aufweist, an welchen eine ein Herausrutschen des Hautfadens (12) aus dem Schlitz (76) behindernde Riffelung (82) ausgebildet ist.

6. Mandrin nach einem der Ansprüche 1 bis 5,
**dadurch gekennzeichnet, dass**
der Grundkörper (18) zumindest bereichsweise aus einem Material gebildet ist, welches eine höhere Wärmeleitfähigkeit aufweist als wenigstens eine weitere Komponente des Mandrins (10), wobei durch ein Beaufschlagen des Materials mit Wärme und/oder durch ein Abführen von Wärme über das Material das Festhalten des Hautfadens (12) mittels des wenigstens einen Halteelements (26) bewirkbar oder zumindest unterstützbar ist.

7. Mandrin nach einem der Ansprüche 1 bis 6,
**dadurch gekennzeichnet, dass**
der Grundkörper (18) hohl ausgebildet ist, wobei
- zumindest im Bereich des wenigstens einen Halteelements (26) wenigstens eine Ansaugöffnung (38) ausgebildet ist, und wobei durch Beaufschlagen der wenigstens einen Ansaugöffnung (38) mit einem Unterdruck ein Festhalten des Hautfadens (12) bewirkbar oder zumindest unterstützbar ist, und/oder
- das wenigstens eine Halteelement (26) ein durch den hohlen Grundkörper (18) hindurchschiebbares Filament (90) umfasst, wobei ein Teilbereich (28) des Hautfadens (12) aufgrund eines Zurückziehens eines aus dem hohlen Grundkörper (18) herausstehenden Endstücks des Filaments (90) in den Grundkörper (18) zwischen einer Innenseite des hohlen Grundkörpers (18) und dem Filament (90) einklemmbar ist.

8. Mandrin nach einem der Ansprüche 1 bis 7,
**dadurch gekennzeichnet, dass**
das wenigstens eine Halteelement (26)
eine, insbesondere eine Mehrzahl von Widerhaken aufweisende, Wendel (68) umfasst, wobei nach einem Fixieren eines freien Endes (70) der Wendel (68) die Wendel (68) durch eine Rotation des Grundkörpers (18) in eine erste Richtung (62) in eine Haltestellung verbringbar ist, in welcher ein Teilbereich (28) des Hautfadens (12) von Windungen (72) der Wendel (68) enger umschließbar ist als in einer Freigabestellung der Wendel (68), in welche die Wendel (68) durch eine Rotation des Grundkörpers (18) in eine zweite Richtung (64) verbringbar ist, welcher der ersten Richtung (62) entgegengesetzt ist.

## Claims

1. A stylet for transplanting a skin filament (12), wherein a base body (18) of the stylet (10) has an inherent rigidity which allows the base body (18) to be pushed through a peripheral venous catheter (14), wherein the stylet (10) has a first end region (16, 24) in which the stylet (10) can be gripped when the stylet (10) is retracted, the first end region (16, 24) providing a stop for pushing the base body (18) through the venous catheter (14), wherein a movement of the base body (18) through the venous catheter (14) in a direction (34) which is opposite to a direction (36) during the pushthrough can be effected by the retraction of the stylet (10), and wherein the stylet (10) has at least one holding element (26) at least in a second end region (20), which is designed to hold the skin filament (12) to be transplanted during the retraction of the basic body (18) through the venous catheter (14), wherein the holding element (26) comprises at least two portions (40, 42) of the base body (18) which are movable away from each other at least in the second end region (20), wherein a partial region (28) of the skin filament (12) which can be arranged between the at least two portions (40, 42) can be clamped due to the retraction of the base body (18) through the venous catheter (14), **characterised in that** the base body (18) is separated over its entire length into the at least two sections (40, 42), which are formed separately from one another and can be moved away from one another to hold the skin filament (12), the at least two separate sections (40, 42) being held together by means of a coupling element (46) of the stylet (10) which can be removed from the base body (18).

2. stylet according to claim 1, **characterised in that** the at least two separate sections (40, 42) are held together in the first end region (16, 24) by means of the coupling element (46) of the stylet (10).

3. stylet according to claim 1 or 2, **characterised in that** in a holding position, in which the clamping of the partial region (28) of the skin filament (12) can be effected by means of the holding element (26), the at least two partial pieces (40, 42) delimit a receiving space (48) formed in the base body (18) and/or the at least two partial pieces (40, 42) in the holding position each have a holding side (50) facing the partial region (28) of the skin filament (12) and an outer side (52) facing away from the holding side (50), wherein a roughness of the holding side (50) is greater than or equal to a roughness of the outer side (52).

4. stylet according to one of claims 1 to 3, **characterised in that** the base body (18), which is designed to taper in particular towards the second end region (20), is designed to be transparent at least in the region of the retaining element (26) and/or at least the retaining element (26) is formed from a material which is different from a material of at least one further subregion of the base body (18).

5. stylet according to one of claims 1 to 4, **characterised in that** the at least one retaining element (26) is provided by a slot (76) formed in the base body (18), in particular in an end region (20) of the base body (18), which slot tapers towards a base (84) of the slot (76) and/or which has mutually opposite side flanks (78, 80) on which a corrugation (82) is formed which prevents the skin filament (12) from slipping out of the slot (76).

6. stylet according to one of claims 1 to 5, **characterised in that** the base body (18) is formed, at least in some regions, from a material which has a higher thermal conductivity than at least one further component of the stylet (10), wherein it is possible to effect or at least support the retention of the skin filament (12) by means of the at least one retaining element (26) by applying heat to the material and/or by dissipating heat via the material.

7. stylet according to one of claims 1 to 6, **characterised in that** the base body (18) is hollow, wherein at least one suction opening (38) is formed at least in the region of the at least one retaining element (26), and wherein by applying a negative pressure to the at least one suction opening (38) it is possible to effect or at least possible to support retention of the skin filament (12), and/or the at least one retaining element (26) comprises a filament (90) which can be pushed through the hollow base body (18), wherein a partial region (28) of the skin filament (12) can be clamped between an inner side of the hollow base body (18) and the filament (90) due to a retraction of an end piece of the filament (90) protruding from the hollow base body (18) into the base body (18).

8. stylet according to one of claims 1 to 7, **characterised in that** the at least one retaining element (26) comprises a helix (68), in particular having a plurality of barbs, wherein, after a free end (70) of the helix (68) has been fixed, the helix (68) can be brought into a holding position by rotating the base body (18) in a first direction (62), in which a partial region (28) of the skin filament (12) can be enclosed more tightly by windings (72) of the helix (68) than in a release position of the helix (68), into which position the helix (68) can be moved by rotating the base body (18) in a second direction (64) which is opposite to the first direction (62).

## Revendications

1. Mandrin pour greffer un fil de peau(12), dans lequel un corps de base (18) du mandrin (10) présente une rigidité propre qui permet de faire passer le corps de base (18) à travers un cathéter veineux périphérique (14), le mandrin (10) présentant une première zone d'extrémité (16, 24) dans laquelle le mandrin (10) peut être saisi lors d'un retrait du mandrin (10), la première zone d'extrémité (16, 24) fournissant une butée pour l'insertion du corps de base (18) à travers le cathéter veineux (14), dans lequel le retrait du mandrin (10) peut provoquer un mouvement du corps de base (18) à travers le cathéter veineux (14) dans une direction (34) qui est opposée à une direction (36) lors de l'insertion, et dans lequel le mandrin (10) présente au moins dans une deuxième zone d'extrémité (20) au moins un élément de retenue (26) qui est conçu pour retenir le fil de peau (12) à transplanter pendant le retrait du corps de base (18) à travers le cathéter veineux (14),
l'élément de retenue (26) comprenant au moins deux pièces partielles (40, 42) du corps de base (18), qui peuvent être éloignées l'une de l'autre au moins dans la deuxième zone d'extrémité (20), une zone partielle (28) du fil de peau (12) pouvant être disposée entre les au moins deux pièces partielles (40, 42) pouvant être coincée à travers le cathéter veineux (14) en raison du retrait du corps de base (18), **caractérisé en ce que** le corps de base (18) est séparé sur toute sa longueur en les au deux pièces partielles (40, 42) qui sont formées séparément l'une de l'autre et qui peuvent être éloignées l'une de l'autre pour retenir le fil de peau (12), les au moins deux pièces partielles (40, 42) séparées l'une de l'autre étant maintenues ensemble au moyen d'un élément de couplage (46) du mandrin (10) pouvant être retiré du corps de base (18).

2. mandrin selon la revendication 1, **caractérisé en ce que** les au moins deux pièces partielles (40, 42) séparées l'une de l'autre sont maintenues ensemble dans la première zone d'extrémité (16, 24) au moyen de l'élément de couplage (46) du mandrin (10).

3. mandrin selon la revendication 1 ou 2, **caractérisé en ce que** les au moins deux pièces partielles (40, 42) délimitent, dans une position de maintien dans laquelle le serrage de la zone partielle (28) du fil de peau (12) peut être effectué au moyen de l'élément de maintien (26), un espace de réception (48) formé dans le corps de base (18) et/ou les au moins deux pièces partielles (40, 42) présentent chacune, dans la position de maintien, un côté de maintien (50) tourné vers la zone partielle (28) du fil de peau (12) et un côté extérieur (52) opposé au côté de maintien (50), une rugosité du côté de maintien (50) étant supérieure ou égale à une rugosité du côté extérieur (52).

4. Mandrin selon l'une quelconque des revendications 1 à 3, **caractérisé en ce que** le corps de base (18), qui se rétrécit en particulier en direction de la deuxième zone d'extrémité (20), est transparent au moins dans la zone de l'élément de retenue (26) et/ou au moins l'élément de retenue (26) est formé d'un matériau qui est différent d'un matériau d'au moins une autre zone partielle du corps de base (18).

5. Mandrin selon l'une des revendications 1 à 4, **caractérisé en ce que** l'au moins un élément de retenue (26) est fourni par une fente (76) formée dans le corps de base (18), en particulier dans une zone d'extrémité (20) du corps de base (18), qui se rétrécit vers un fond (84) de la fente (76) et/ou qui présente des flancs latéraux (78, 80) opposés l'un à l'autre, sur lesquels est formée une cannelure (82) empêchant le fil de peau (12) de glisser hors de la fente (76).

6. Mandrin selon l'une des revendications 1 à 5, **caractérisé en ce que** le corps de base (18) est formé au moins par zones d'un matériau qui présente une conductivité thermique plus élevée qu'au moins un autre composant du mandrin (10), la fixation du fil de peau (12) au moyen de l'au moins un élément de retenue (26) pouvant être provoquée ou au moins soutenue par une exposition du matériau à la chaleur et/ou par une évacuation de la chaleur par le biais du matériau.

7. Mandrin selon l'une quelconque des revendications 1 à 6, **caractérisé en ce que** le corps de base (18) est creux, dans lequel au moins une ouverture d'aspiration (38) est formée au moins dans la zone de l'au moins un élément de retenue (26), et dans lequel, en soumettant l'au moins une ouverture d'aspiration (38) à une dépression, il est possible de provoquer ou au moins de soutenir un maintien du fil de peau (12), et/ou l'au moins un élément de maintien (26) comprend un filament (90) pouvant être poussé à travers le corps de base creux (18), une zone partielle (28) du fil de peau (12) pouvant être coincée dans le corps de base (18) entre un côté intérieur du corps de base creux (18) et le filament (90) en raison d'un retrait d'une pièce d'extrémité du filament (90) dépassant du corps de base creux (18).

8. Mandrin selon l'une quelconque des revendications 1 à 7, **caractérisé en ce que** l'au moins un élément de retenue (26) comprend une hélice (68) présentant en particulier une pluralité de barbes, l'hélice (68) pouvant être amenée dans une position de retenue par une rotation du corps de base (18) dans une première direction (62) après une fixation d'une extrémité libre (70) de l'hélice (68), dans laquelle une zone partielle (28) du fil de peau (12) peut être entourée plus étroitement par des spires (72) de l'hélice (68) que dans une position de libération de l'hélice (68), dans laquelle l'hélice (68) peut être amenée par une rotation du corps de base (18) dans une deuxième direction (64), qui est opposée à la première direction (62).
